# EUROPÄISCHE PATENTSCHRIFT

(11) **EP 2 373 233 B1**
(45) Veröffentlichungstag und Bekanntmachung des Hinweises auf die Patenterteilung: **13.07.2016**
(21) Anmeldenummer: 09737356.7
(22) Anmeldetag: 14.10.2009
(51) Int. Cl.: A61B 17/28

(54) **HANDGRIFFVORRICHTUNG**
HANDLE DEVICE
DISPOSITIF DE POIGNÉE

(30) Priorität: 19.11.2008 DE 102008058207
(43) Veröffentlichungstag der Anmeldung: 12.10.2011
(73) Patentinhaber: Dan&Med Repair Dannoritzer Medizintechnik Gmbh&Co. KG, 78532 Tuttlingen (DE)
(72) Erfinder: HAUSER, Michael, 78559 Gosheim (DE)
(74) Vertreter: Daub, Thomas
(86) Internationale Anmeldenummer: PCT/EP2009/007363
(87) Internationale Veröffentlichungsnummer: WO 2010/057551

(56) Entgegenhaltungen:
- WO-A1-94/00059
- WO-A1-96/03930
- WO-A2-98/11814
- FR-A- 842 399
- US-A- 2 530 136
- US-A- 2 632 661
- US-A- 4 611 595
- US-A- 5 755 726

## Beschreibung

### Stand der Technik

Die Erfindung geht aus von einer Handgriffvorrichtung nach dem Oberbegriff des Anspruchs 1.

Es sind bereits Handgriffvorrichtungen, insbesondere für Chirurgiewerkzeuge, mit zwei Griffelementen und einem Scherengelenk, mittels dessen die zwei Griffelemente schwenkbar verbunden sind, sowie mit einer Kopplungseinheit, die dazu vorgesehen ist, die beiden Griffelemente zusammenzuhalten, bekannt. Die Kopplungseinheit wird dabei von einer Schraube oder einer Niete Haltegriff gebildet.

Dokument DE 20 2007 000 427 offenbart eine Chirurgischewerkzeughandgriffvorrichtung gemäss dem Oberbegriff des Anspruchs 1.

### Vorteile der Erfindung

Die Erfindung geht aus von einer Handgriffvorrichtung, insbesondere einer Chirurgiewerkzeug-Handgriffvorrichtung, mit einem ersten und zumindest einem zweiten Griffelement und einem Scherengelenk, mittels dessen die zwei Griffelemente schwenkbar verbunden sind, sowie mit einer Kopplungseinheit, die dazu vorgesehen ist, die beiden Griffelemente zusammenzuhalten.

Unter einem "Scherengelenk" soll in diesem Zusammenhang insbesondere eine mechanische Verbindung der Griffelemente verstanden werden, die eine relative Drehbewegung der beiden Griffelemente um eine gemeinsame Drehachse, die senkrecht zu der von den beiden Griffelementen aufgespannten Ebene und innerhalb ihrer Verbindung liegt, ermöglicht.

Es wird vorgeschlagen, dass die Kopplungseinheit wenigstens ein Kopplungselement aufweist, das dazu vorgesehen ist, abhängig von einer Relativposition der Griffelemente eine Formschlussverbindung derselben herzustellen. Durch eine entsprechende Ausgestaltung kann eine besonders einfache, insbesondere werkzeuglose Montage der Griffelemente erreicht werden.

Des Weiteren wird vorgeschlagen, dass die Kopplungseinheit dazu vorgesehen ist, abhängig von einer Relativposition der Griffelemente eine reversible Formschlussverbindung derselben herzustellen. Unter einer "reversiblen" Formschlussverbindung soll in diesem Zusammenhang insbesondere eine Formschlussverbindung verstanden werden, deren Lösung durch eine Umkehr der Montagereihenfolge erfolgt, wodurch vorteilhaft eine besonders einfache Montage und Demontage erreicht werden kann. Vorteilhaft sind die Kopplungselemente in Form von Merkmalen ausgestaltet, die sich nach dem Schlüssel-Schloss-Prinzip bei einer Überdeckung komplementär ergänzen, wobei die Kopplungselemente der Griffelemente im Verlauf der Herstellung der Formschlussverbindung aneinander vorbei geführt werden können und die Herstellung des Formschlusses durch eine translatorische, eine rotatorische oder eine Kombination beider Bewegungen der Kopplungselemente erfolgen kann. In einer weiteren Ausgestaltung kann durch die Auswahl und Anordnung der Merkmale vorteilhaft eine Zuordnungsrelation von bestimmten Griffelementen oder eine Ausschlussrelation für eine gemeinsame Verwendung von bestimmten Griffelementen hergestellt werden.

Insbesondere kann in einer vorteilhaften Ausgestaltung die Bewegung zur Herstellung der Formschlussverbindung der beiden Griffelemente differierend von einer relativen Drehbewegung der beiden Griffelemente während einer normalen Bedienung der Handgriffvorrichtung gewählt sein. Besonders vorteilhaft kann in einer weiteren Ausgestaltung eine Schwenkbewegung eines der Griffelemente zur Herstellung der Formschlussverbindung so ausgelegt sein, dass die Schwenkbewegung in einer Ebene stattfindet, die in einem Winkelbereich von bis zu 30° zu beiden Seiten einer Ebene liegt, die senkrecht zu der von den beiden Griffelementen während der normalen Bedienung der Handgriffvorrichtung aufgespannten Ebene verläuft, so dass die Formschlussverbindung bei normalem Betrieb gesichert ist und keine Entkopplung der Griffelemente erfolgen kann.

Ferner wird vorgeschlagen, dass die Formschlussverbindung wenigstens ein Kopplungselement aufweist, das zumindest teilweise einstückig mit einem Griffelement ausgebildet ist. Die Handgriffvorrichtung kann auf einfache Weise ohne die Demontage zusätzlicher Teile in seine Bestandteile zerlegt werden, wodurch insbesondere im Fall von Handgriffvorrichtungen für Chirurgiewerkzeuge in vorteilhafter Weise eine Eignung für eine Dampfdrucksterilisation (Autoklavierbarkeit) erreicht werden kann.

Weiterhin wird vorgeschlagen, dass zumindest das erste und das zweite Griffelement jeweils Zentrierelemente umfassen, die zur Zentrierung der Formschlussverbindung vorgesehen sind, wodurch eine vorteilhaft präzise Führung der relativen Drehbewegung der Griffelemente erzielt werden kann. Insbesondere kann in einer Ausgestaltung der Zentrierelemente deren Symmetrieachse vorteilhaft parallel zur Drehachse des Scherengelenks liegen.

Ferner wird vorgeschlagen, dass die Handgriffvorrichtung eine Werkzeugeinheit umfasst, die zumindest teilweise einen mechanischen Anschlag für die Kopplungseinheit bildet, wodurch der Schwenkbereich des Scherengelenks mechanisch begrenzt und vorteilhaft eine Einsparung von zusätzlichen Bauteilen zur Herstellung dieser Funktion erreicht werden kann. Unter dem "Schwenkbereich" des Scherengelenks sollen in diesem Zusammenhang insbesondere die Sektorwinkel verstanden werden, die während einer maximal möglichen relativen Bewegung von den Griffelementen überstrichen werden.

Umfasst die Werkzeugeinheit wie vorgeschlagen eine Werkzeughülse, in der ein Werkzeug beweglich gelagert ist, kann eine besonders gute Führung und Positionierung des Werkzeuges erzielt werden.

Es wird vorgeschlagen, dass zumindest ein Griffelement ein Antriebselement für einen translatorischen Antrieb des Werkzeugs umfasst, wodurch vorteilhaft eine relative Drehbewegung der Griffelemente in eine translatorische Bewegung des Werkzeugs umgesetzt werden kann.

Weiterhin wird vorgeschlagen, dass zumindest eines der Griffelemente eine Vorrichtung zur mechanischen Sicherung einer Werkzeugeinheit umfasst, so dass vorteilhaft ein Austausch der Werkzeugeinheit möglich ist und die Handgriffvorrichtung und die Werkzeugeinheit auf vorteilhaft einfache Weise fest verbunden werden können.

Ferner wird vorgeschlagen, dass die Handgriffvorrichtung ein Sicherungselement aufweist, das an wenigstens einem der Griffelemente angeordnet ist und das dazu vorgesehen ist, während der Bedienung des Werkzeugs die Formschlussverbindung zu sichern. Insbesondere kann in einer vorteilhaften Ausgestaltung des Sicherungselements eine Ausrichtung der Griffelemente entsprechend der für die Herstellung oder eine Lösung der Formschlussverbindung notwendigen Relativposition verhindert werden.

Es wird vorgeschlagen, dass das an wenigstens einem der Griffelemente angeordnete Sicherungselement eine Sicherungsstellung und eine Freigabestellung aufweist. Insbesondere kann in einer vorteilhaften Ausführung wahlweise zwischen der Sicherungsstellung zur Sicherung der Formschlussverbindung und der Freigabestellung zur Vorbereitung einer Lösung der Formschlussverbindung gewechselt werden.

Es wird vorgeschlagen, dass das Kopplungselement dazu vorgesehen ist, die Herstellung der Formschlussverbindung der beiden Griffelemente in einer Relativposition zwischen den Griffelementen herzustellen, die einer Schließstellung der Griffelemente entspricht. Unter der "Schließstellung" der Griffelemente soll in diesem Zusammenhang insbesondere diejenige Stellung der Griffelemente verstanden werden, bei der diese den minimal möglichen Sektorwinkel einschließen. Insbesondere kann in einer geeigneten Ausgestaltung in Verbindung mit dem Sicherungselement vorteilhaft sichergestellt werden, dass ein Lösen der Formschlussverbindung während der Betätigung der Handgriffvorrichtung ausgeschlossen ist.

Weiterhin wird vorgeschlagen, dass die Kopplungseinheit mindestens ein Begrenzungselement aufweist, das dazu vorgesehen ist, einen Bereich für die möglichen Relativpositionen der Griffelemente festzulegen, wodurch vorteilhaft der Bewegungsbereich des Werkzeugs präzise definiert werden kann.

Vorschlagsgemäß weist die Kopplungseinheit mindestens ein Begrenzungselement auf, das dazu vorgesehen ist, bei einer definierten relativen Stellung der Griffelemente deren Bewegung mechanisch zu blockieren, wodurch besonders vorteilhaft unerwünschte Stellungen des Werkzeugs vermieden werden können. In einer weiteren vorteilhaften Ausgestaltung kann beim Einsatz von zangenartigen Werkzeugen durch ein bewusstes Auslösen der Blockierung vorteilhaft eine Selbsthaltefunktion des Werkzeugs erzielt werden.

Es wird des Weiteren vorgeschlagen, dass zumindest eines der Griffelemente ein Aufnahmeelement aufweist und zumindest ein weiteres der Griffelemente ein Begrenzungselement zur Begrenzung einer Aufspreizung des Scherengelenks aufweist, wodurch vorteilhaft eine bessere Handhabung des Werkzeugs erreicht werden kann. In einer vorteilhaften Ausgestaltung kann beim Erreichen einer festgelegten Größe des Sektorwinkels des Scherengelenks durch ein Zusammenwirken des Begrenzungselements und des Aufnahmeelements eine genau definierte Endstellung erzielt werden.

### Zeichnung

Weitere Vorteile ergeben sich aus der folgenden Zeichnungsbeschreibung. In den Zeichnungen ist ein Ausführungsbeispiel der Erfindung dargestellt. Die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Handgriffvorrichtung für ein Chirurgiewerkzeug mit zwei Griffelementen und einer Werkzeugeinheit in Bedienstellung,
- Fig. 2: einen Teil der Kopplungseinheit in einem Griffelement zur Aufnahme der Werkzeugeinheit (ohne Werkzeugeinheit),
- Fig. 3: den anderen Teil der Kopplungseinheit in dem anderen Griffelement,
- Fig. 4: einen Schnitt durch die Handgriffvorrichtung im Bereich der Kopplungseinheit,
- Fig. 5: die Stellung der Griffelemente in der Schließstellung und
- Fig. 6: die Stellung der Griffelemente bei der maximalen Aufspreizung.

### Beschreibung des Ausführungsbeispiels

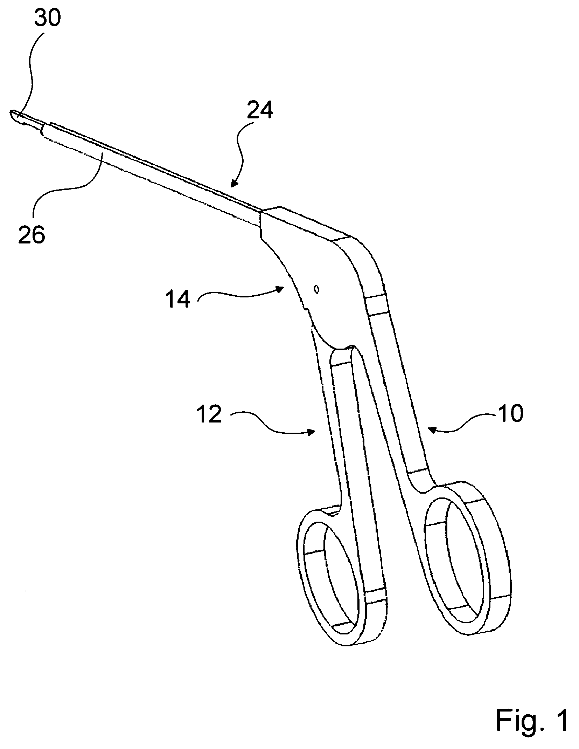

Fig. 1 zeigt eine Handgriffvorrichtung für ein Chirurgiewerkzeug mit zwei Griffelementen 10 und 12 und einem Scherengelenk, mittels dessen die zwei Griffelemente 10 und 12 schwenkbar verbunden sind, zur Bedienung eines Werkzeuges 30, das, in einer Werkzeughülse 26 beweglich gelagert, mit dieser eine Werkzeugeinheit 24 bildet.

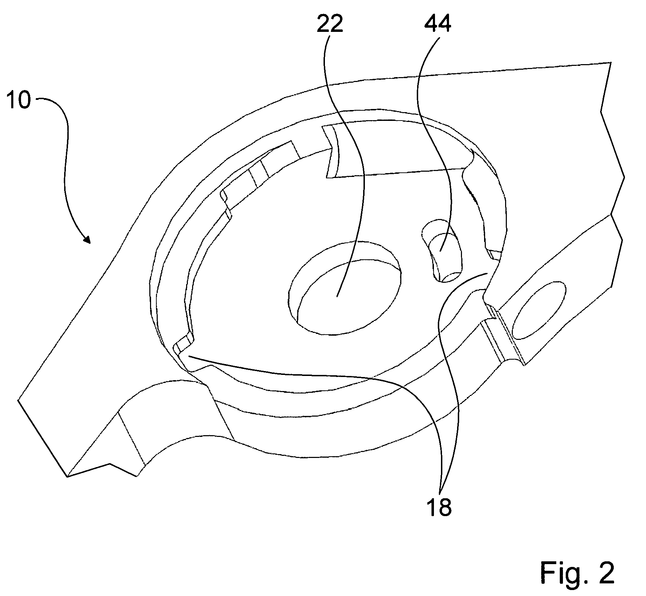

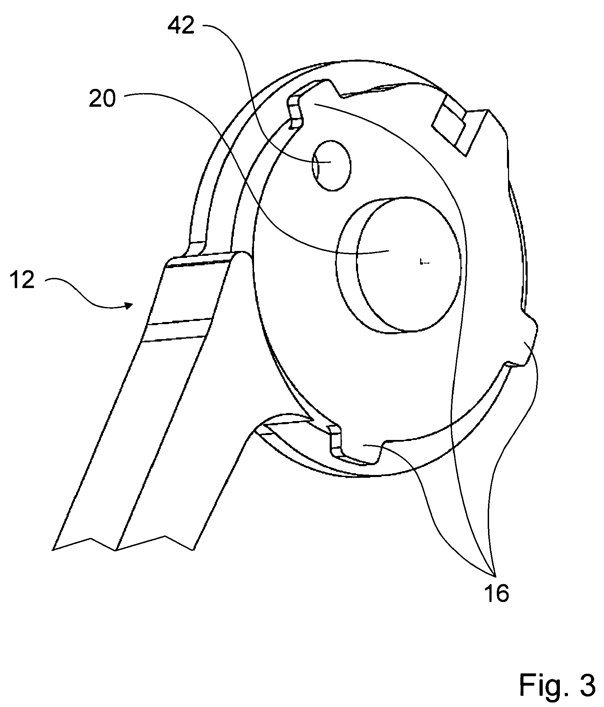

Die in den Fig. 2 und 3 dargestellte Kopplungseinheit 14 ist dazu vorgesehen, abhängig von einer Relativposition der Griffelemente 10 und 12 eine Formschlussverbindung derselben herzustellen. Diese Formschlussverbindung kann hergestellt werden, indem drei nockenförmig und einstückig mit dem Griffelement 12 ausgebildete, auf einem Kreisring um eine Drehachse angeordnete Kopplungselemente 16 (Fig. 3) an den entsprechenden Kopplungselementen 18 in Form von Aussparungen in dem anderen Griffelement 10 (Fig. 2), ebenfalls einstückig mit diesem ausgebildet, vorbei geschoben werden. Bei einer relativen Drehbewegung der beiden Griffelemente 10 und 12 gegeneinander führen die nockenförmigen Kopplungselemente 16 eine kreisförmige Bewegung innerhalb einer hinter den Kopplungselementen 18 in Form von Aussparungen angelegten Nut aus. Die Formschlussverbindung der Griffelemente 10 und 12 ist reversibel ausgeführt und durch eine Umkehrung der Montagereihenfolge lösbar.

Die zur Zentrierung der Formschlussverbindung vorgesehenen Zentrierelemente in den beiden Griffelementen 10 und 12 sind zum einen als konusförmiges Zentrierelement 20 in Form eines Zapfens im Zentrum des Scherengelenks und zum anderen als ein zur Aufnahme des Zapfens dienendes, als Aussparung ausgeformtes Zentrierelement 22 ausgestaltet, wobei die Symmetrieachse der Zentrierelemente parallel zur Drehachse des Scherengelenks liegt.

Die Kopplungseinheit umfasst Begrenzungselemente, die dazu vorgesehen sind, einen Bereich für die möglichen Relativpositionen der Griffelemente 10 und 12 festzulegen. Im Griffelement 10 ist das Begrenzungselement 44 als eine Ausnehmung in radialer Richtung der Kopplungseinheit mit einem Durchgangsloch an seinem Ende (Fig. 2) ausgeformt. Im Griffelement 12 befindet sich an der entsprechenden Position das Begrenzungselement 42 in Form eines in einem Sackloch federnd gelagerten Führungszapfens (Fig. 3). Bei der Herstellung der Formschlussverbindung kann der Führungszapfen sich innerhalb der Führung der Ausnehmung bewegen, wodurch der Bereich für die möglichen Relativpositionen der Griffelemente 10 und 12 festgelegt wird. Bei einer definierten relativen Stellung der Griffelemente 10 und 12, nämlich bei Erreichen des Durchgangslochs im Begrenzungselement 44, wird durch den in das Durchgangsloch vorspringenden Führungszapfen eine weitere Bewegung der Griffelemente 10 und 12 mechanisch blockiert.

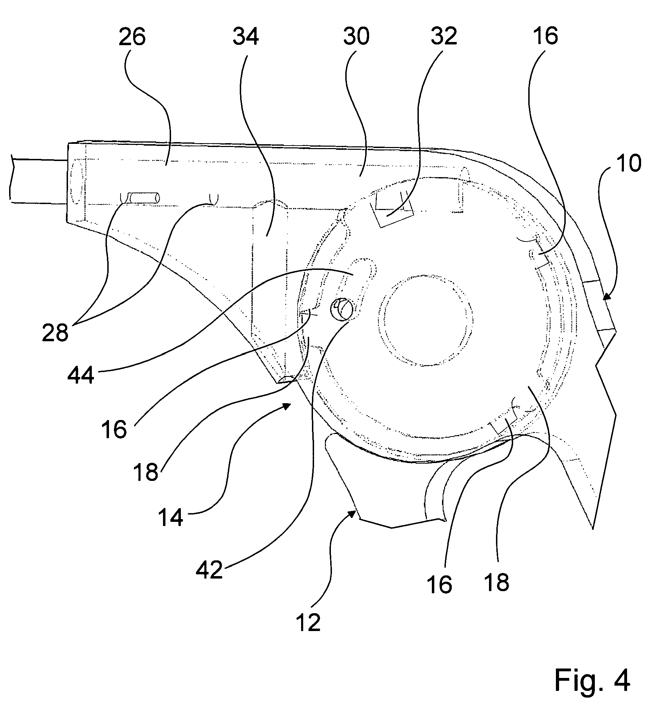

In der Fig. 4 ist ein als Aussparung in einem Drehkranz innerhalb des Scherengelenks ausgestaltetes Antriebselement 32 für einen translatorischen Antrieb des Werkzeugs 30 dargestellt. Das Ende des Werkzeugs 30 ragt in das als Aussparung ausgeformte Antriebselement 32 hinein und wird bei einer relativen Drehung der Griffelemente 10 und 12 mitgenommen. Die Werkzeugeinheit 24 umfasst wie dargestellt an der Werkzeughülse 26 einen mechanischen Anschlag 28 für die Kopplungseinheit 14. Das Griffelement 10 umfasst als Sicherungsvorrichtung 34 eine zur mechanischen Sicherung der Werkzeugeinheit 24 ausgeführte Sicherungsschraube.

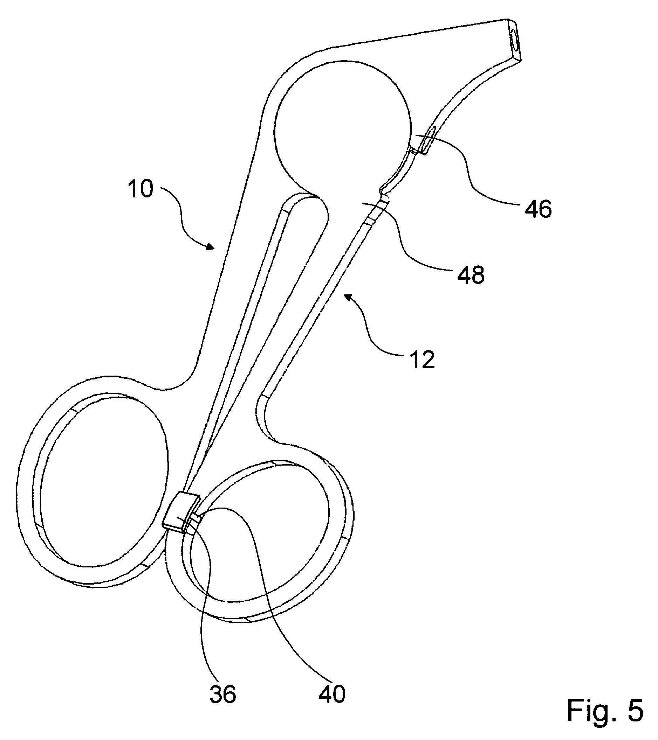

Fig. 5 zeigt die beiden Griffelemente 10 und 12 in ihrer Schließstellung, in der die Herstellung bzw. Lösung der Formschlussverbindung der Griffelemente 10 und 12 vorgenommen werden kann.

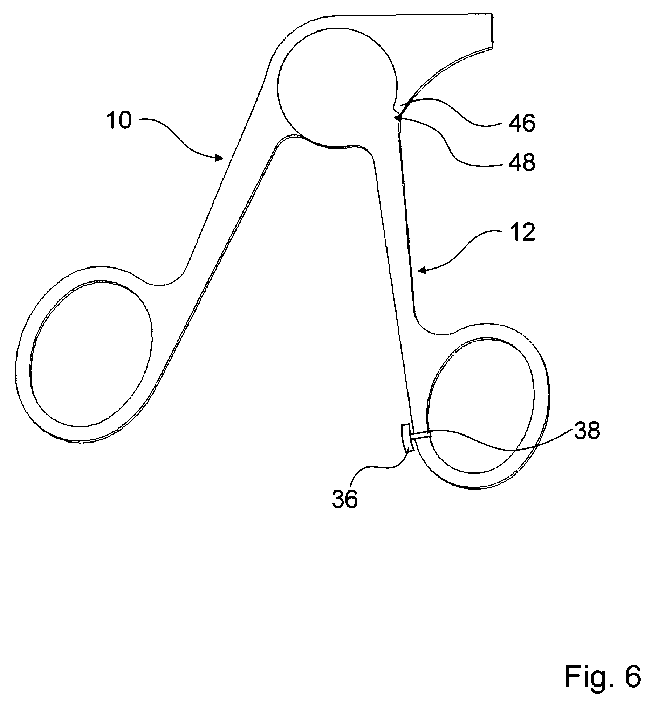

In der Fig. 6 sind die beiden Griffelemente 10 und 12 in einer Aufspreizung des Scherengelenks dargestellt, bei der das Aufnahmeelement 46 des einen Griffelements 10 im Zusammenwirken mit dem Begrenzungselement 48 des anderen Griffelements 12 den maximalen Sektorwinkel für eine Aufspreizung des Scherengelenks begrenzt.

In den Fig. 5 und 6 ist ein am Griffelement 12 angeordnetes Sicherungselement 36 dargestellt, das dazu vorgesehen ist, während der Bedienung des Werkzeugs 30 die Formschlussverbindung der Griffelemente zu sichern. In der Fig. 5 ist das Sicherungselement 36 in der Freigabestellung 40 dargestellt; Fig. 6 zeigt das Sicherungselement 36 in der Sicherungsstellung 38.

### Bezugszeichen

- 10: Griffelement
- 12: Griffelement
- 14: Kopplungseinheit
- 16: Kopplungselement
- 18: Kopplungselement
- 20: Zentrierelement
- 22: Zentrierelement
- 24: Werkzeugeinheit
- 26: Werkzeughülse
- 28: Anschlag
- 30: Werkzeug
- 32: Antriebselement
- 34: Sicherungsvorrichtung
- 36: Sicherungselement
- 38: Sicherungsstellung
- 40: Freigabestellung
- 42: Begrenzungselement
- 44: Begrenzungselement
- 46: Aufnahmeelement
- 48: Begrenzungselement

## Patentansprüche

1. Chirurgiewerkzeug-Handgriffvorrichtung mit einem ersten und zumindest einem zweiten Griffelement (10, 12) und einem Scherengelenk, mittels dessen die zwei Griffelemente (10, 12) schwenkbar verbunden sind, sowie mit einer Kopplungseinheit (14), die dazu vorgesehen ist, die beiden Griffelemente (10, 12) zusammenzuhalten, wobei die Kopplungseinheit (14) wenigstens ein Kopplungselement (16, 18) aufweist, das dazu vorgesehen ist, abhängig von einer Relativposition der Griffelemente (10, 12) eine Formschlussverbindung der Griffelemente (10, 12) herzustellen, wobei die Formschlussverbindung wenigstens das Kopplungselement (16, 18) aufweist, das einstückig mit einem der Griffelemente (10, 12) ausgebildet ist, und mit einer Werkzeugeinheit (24), die eine Werkzeughülse (26) umfasst, in der ein Werkzeug (30) beweglich gelagert ist, wobei zumindest eines der Griffelemente (10, 12) ein Antriebselement (32) für einen translatorischen Antrieb des Werkzeugs (30) umfasst, wobei zumindest eines der Griffelemente (10, 12) eine Sicherungsvorrichtung (34) zu einer mechanischen Sicherung der Werkzeugeinheit (24) umfasst, **dadurch gekennzeichnet, dass**
zumindest das erste und das zweite Griffelement (10, 12) jeweils Zentrierelemente (20,22) umfassen, die zur Zentrierung der Formschlussverbindung vorgesehen sind, wobei die Werkzeugeinheit (24) zumindest teilweise einen mechanischen Anschlag (28) für die Kopplungseinheit (14) bildet.

2. Handgriffvorrichtung nach Anspruch 1,
**dadurch gekennzeichnet, dass**
die Kopplungseinheit (14) dazu vorgesehen ist, abhängig von einer Relativposition der Griffelemente (10, 12) eine reversible Formschlussverbindung derselben herzustellen.

3. Handgriffvorrichtung nach einem der vorhergehenden Ansprüche,
**gekennzeichnet durch**
ein Sicherungselement (36), das an wenigstens einem der Griffelemente (10, 12) angeordnet ist und das dazu vorgesehen ist, während der Bedienung des Werkzeugs (30) die Formschlussverbindung zu sichern.

4. Handgriffvorrichtung nach Anspruch 3,
**dadurch gekennzeichnet, dass**
das an wenigstens einem der Griffelemente (10, 12) angeordnete Sicherungselement (36) eine Sicherungsstellung (38) und eine Freigabestellung (40) aufweist.

5. Handgriffvorrichtung nach einem der vorhergehenden Ansprüche,
**dadurch gekennzeichnet, dass**
die Kopplungseinheit (14) dazu vorgesehen ist, die Herstellung der Formschlussverbindung der beiden Griffelemente (10, 12) in einer Relativposition zwischen den Griffelementen (10, 12) herzustellen, die einer Schließstellung der Griffelemente (10, 12) entspricht.

6. Handgriffvorrichtung nach einem der vorhergehenden Ansprüche,
**dadurch gekennzeichnet, dass**
die Kopplungseinheit (14) mindestens ein Begrenzungselement (42, 44) aufweist, das dazu vorgesehen ist, einen Bereich für die möglichen Relativpositionen der Griffelemente (10, 12) festzulegen.

7. Handgriffvorrichtung nach einem der vorhergehenden Ansprüche,
**dadurch gekennzeichnet, dass**
die Kopplungseinheit (14) mindestens ein Begrenzungselement (42, 44) aufweist, das dazu vorgesehen ist, bei einer definierten relativen Stellung der Griffelemente (10, 12) deren Bewegung mechanisch zu blockieren.

8. Handgriffvorrichtung nach einem der vorhergehenden Ansprüche,
**dadurch gekennzeichnet, dass**
zumindest eines der Griffelemente (10, 12) ein Aufnahmeelement (46) aufweist und zumindest ein weiteres der Griffelemente (10, 12) ein Begrenzungselement (48) zur Begrenzung einer Aufspreizung des Scherengelenks aufweist.

## Claims

1. Handle device of a surgical instrument, with a first and at least one second handle element (10, 12) and with a scissors joint via which the two handle elements (10, 12) are pivotably connected, and with a coupling unit (14) which is provided for holding the two handle elements (10, 12) together, the coupling unit (14) comprising at least one coupling element (16, 18), which is provided to establish a form-fit connection of the handle elements (10, 12) depending on a relative position of the handle elements (10, 12), the form-fit connection comprising at least the coupling element (16, 18), which is embodied in a one-part implementation with one of the handle elements (10, 12), and with a tool unit (24) comprising a tool sleeve (26) in which a tool (30) is movably supported, at least one of the handle elements (10, 12) comprising a drive element (32) for translationally driving the tool (30), at least one of the handle elements (10, 12) comprising a securing device (34) for mechanically securing the tool unit (24),
**characterised in that**
at least the first and the second handle element (10, 12) respectively comprise centering elements (20, 22), which are provided for centering the form-fit connection, the tool unit (24) at least partially forming a mechanical stop (28) for the coupling unit (14).

2. Handle device according to claim 1,
**characterised in that**
the coupling unit (14) is provided to establish a reversible form-fit connection of the handle elements (10, 12) depending on a relative position of said handle elements (10, 12).

3. Handle device according to one of the preceding claims,
**characterised by**
a securing element (36) which is arranged on at least one of the handle elements (10, 12) and is provided for securing the form-fit connection during operation of the tool (30).

4. Handle device according to claim 3,
**characterised in that**
the securing element (36) arranged on at least one of the handle elements (10, 12) has a securing position (38) and a release position (40).

5. Handle device according to one of the preceding claims,
**characterised in that**
the coupling unit (14) is provided for establishing a form-fit connection of the two handle elements (10, 12) in a relative position between the handle elements (10, 12) that corresponds to a closure position of the handle elements (10, 12).

6. Handle device according to one of the preceding claims,
**characterised in that**
the coupling unit (14) comprises at least one delimiting element (42, 44), which is provided for determining a range for the possible relative positions of the handle elements (10, 12).

7. Handle device according to one of the preceding claims,
**characterised in that**
the coupling unit (14) comprises at least one delimiting element (42, 44), which is provided for mechanically blocking a movement of the handle elements (10, 12) in a defined relative position of said handle elements (10, 12).

8. Handle device according to one of the preceding claims,
**characterised in that**
at least one of the handle elements (10, 12) comprises a receiving element (46) and at least one further one of the handle elements (10, 12) comprises a delimiting element (48) for delimiting a spreading of the scissors joint.

## Revendications

1. Dispositif de poignée d'outil chirurgical, avec un premier et au moins un deuxième élément de poignée (10, 12) et avec une articulation à ciseaux, par le biais de laquelle les deux éléments de poignée (10, 12) sont joints pivotablement, et avec une unité de couplage (14) prévue à tenir ensemble les deux éléments de poignée (10, 12), l'unité de couplage (14) comportant au moins un élément de couplage (16, 18) prévu à établir une connexion par forme des éléments de poignée (10, 12) en dépendance à une position relative des éléments de poignée (10, 12), la connexion par forme comportant au moins un élément de couplage (16, 18) implémenté d'une part avec un des éléments de poignée (10, 12), et avec une unité d'outil (24) comportant un manchon d'outil (26), dans lequel un outil (30) est supporté mouvablement, au moins un des éléments de poignée (10, 12) comprenant un élément d'entraînement (32) pour un entraînement translationnel de l'outil (30), au moins un des éléments de poignée (10, 12) comprenant un dispositif d'assurage (34) pour un assurage mécanique de l'unité d'outil (24), **caractérisé en ce qu'**
au moins le premier et le deuxième élément de poignée (10, 12) respectivement comportent des éléments de centrage (20, 22) prévus au centrage de la connexion par forme, l'unité d'outil (24) formant au moins partiellement un arrêt (28) mécanique pour l'unité de couplage (14).

2. Dispositif de poignée selon la revendication 1,
**caractérisé en ce que**
l'unité de couplage (14) est prévue à établir une connexion par forme réversible des éléments de poignée (10, 12) en dépendance à une position relative desdits éléments de poignée (10, 12).

3. Dispositif de poignée selon l'une quelconque des revendications précédentes, **caractérisé par**
un élément d'assurage (36) disposé à au moins un des éléments de poignée (10, 12) et prévu à assurer la connexion par forme pendant l'opération de l'outil (30).

4. Dispositif de poignée selon la revendication 3,
**caractérisé en ce que**
l'élément d'assurage (36) disposé à au moins un des éléments de poignée (10, 12) comporte une position d'assurage (38) et une position de déblocage (40).

5. Dispositif de poignée selon l'une quelconque des revendications précédentes, **caractérisé en ce que**
l'unité de couplage (14) est prévue à établir la connexion par forme des deux éléments de poignée (10, 12) dans une position relative entre les éléments de poignée (10, 12) correspondante à une position de fermeture des éléments de poignée (10, 12).

6. Dispositif de poignée selon l'une quelconque des revendications précédentes, **caractérisé en ce que**
l'unité de couplage (14) comporte au moins un élément de limitation (42, 44) prévu à déterminer une portée pour les positions relatives possibles des éléments de poignée (10, 12).

7. Dispositif de poignée selon l'une quelconque des revendications précédentes, **caractérisé en ce que**
l'unité de couplage (14) comporte au moins un élément de limitation (42, 44) prévu à mécaniquement bloquer le mouvements des éléments de poignée (10, 12) dans une position relative définie desdits éléments de poignée (10, 12).

8. Dispositif de poignée selon l'une quelconque des revendications précédentes, **caractérisé en ce qu'**
au moins un des éléments de poignée (10, 12) comporte un élément récepteur (46) et au moins un autre des éléments de poignée (10, 12) comporte un élément de limitation (48) pour limiter un écartement de l'articulation à ciseaux.
